# EUROPEAN PATENT APPLICATION

(11) **EP 3 384 850 A1**
(43) Date of publication of application: **10.10.2018**
(21) Application number: 17165053.4
(22) Date of filing: 05.04.2017
(51) Int. Cl.: A61B 8/06, A61B 8/08

(54) **METHOD AND APPARATUS FOR PHYSIOLOGICAL FUNCTIONAL PARAMETER DETERMINATION**

(71) Applicant: Koninklijke Philips N.V., 5656 AE Eindhoven (NL)
(72) Inventor: NICKISCH, Hannes, 5656 AE Eindhoven (NL); GRASS, Michael, 5656 AE Eindhoven (NL); HAASE, Christian, 5656 AE Eindhoven (NL); SCHMITT, Holger, 5656 AE Eindhoven (NL)
(74) Representative: de Haan, Poul Erik

(57) **Abstract**

The present invention relates to a determination of a physiological functional parameter of a living being. Ultrasound image data and Doppler image data of a vessel structure are provided (101) and registered (102). The vessel structure is segmented (103) to generate (104) a representation of the vessel structure. The flow velocity inside a vessel of the vessel structure is determined (105) based on the Doppler image data. A physiological functional parameter determination model defining a value of a functional physiological parameter in dependence of a representation of a vessel structure and a flow velocity inside a vessel of the vessel structure is used (106) to determine (107) the physiological functional parameter inside the vessel of the vessel structure. The representation of the vessel structure and/or the flow velocity values can be constantly updated upon receipt of further input images to provide an estimation of the functional physiological parameter in real-time.

## Description

### FIELD OF THE INVENTION

The present invention relates to an apparatus, a method and a computer program for determining of a physiological functional parameter of a living being.

### BACKGROUND OF THE INVENTION

Functional physiological parameters such as the fractional flow reserve (FFR) are an important predictor of vascular health. They are typically measured invasively. Alternatively, the fluid dynamics within the vascular system can be simulated based on a computed tomography (CT) image of the vascular system, in order to determine the FFR value. However, a patient is exposed to ionizing radiation during computed tomography imaging, which can harm the patient.

### SUMMARY OF THE INVENTION

It is an object of the present invention to provide an apparatus, a method and a computer program for determining one or more physiological functional parameters, in particular an intravascular pressure gradient or a peripheral FFR, of a living being in a manner being harmless for the health of the living being.

The object is achieved by the apparatus, method and computer program according to the independent claims. All the local anatomical and functional information comprised in ultrasound image data is incorporated into a comprehensive functional patient model allowing to predict physiological functional parameters such as pressure gradients or (peripheral) FFRs without requiring patient data obtained by using ionizing radiation.

In an aspect of the present invention an apparatus for determining a physiological functional parameter of a living being is presented, wherein the apparatus comprises:
an image providing unit for providing ultrasound image data and Doppler image data of a vessel structure of the living being;
a registration unit for registering the ultrasound image data and the Doppler image data;
a segmentation unit for segmenting the vessel structure in the ultrasound image data, thereby generating a vessel structure segmentation;
a representation generation unit for generating a representation of the vessel structure based on the vessel structure segmentation;
a flow velocity value determination unit for determining flow velocity values inside a vessel of the vessel structure based on the Doppler image data; and
a physiological functional parameter determination unit for determining the physiological functional parameter of the living being, wherein the physiological functional parameter determination unit is adapted to a) provide a functional parameter determination model defining a functional physiological parameter in dependence of a representation of a vessel structure and flow velocity values inside a vessel of the vessel structure and b) determine the physiological functional parameter by using the functional parameter determination model, the generated representation of the vessel structure and the determined flow velocity values.

The image providing unit provides ultrasound image data and Doppler image data of a vessel structure, wherein the vessel structured covered by the Doppler image data is also covered by the ultrasound image data. The image providing unit may provide the ultrasound image data and the Doppler image data by receiving live image data directly from an ultrasound probe which is positioned over a patient's skin. The image providing unit may also provide the image data from a local memory or a remote server, e.g. image data of a previous session etc. The Doppler image data may be generated from ultrasound Doppler data recorded upon explicit command or automatically with the pure ultrasound image data. The Doppler image data do not have to be provided for each and every position within the ultrasound image data. A sonographer may activate Doppler image data taking for instance by pressing a particular button and thus control the respective regions in which ultrasound Doppler image data is taken in addition to pure ultrasound image data.

The ultrasound image preferentially include several ultrasound images for several times and/or for several phases, especially for different cardiac phases. Correspondingly, also the Doppler image data preferentially include several Doppler images for different times and/or for different phases. The registration unit may be adapted to temporally and spatially align the ultrasound image data and the Doppler image data. The registration unit may use information extracted from the image data itself and/or external data, like timestamps (relative to the cardiac cycle) and position data if available. The registration unit may for instance extract information from the image data by using local image features such as speckles, vector flow images or anatomy which may guide the alignment via a similarity measure e.g. correlation. As pointed out herein above images taken for one and the same position of an ultrasound probe over the skin of a living being do not necessarily show a static image. Across a cardiac cycle, the vessel structure might show some movements. Thus, temporal and spatial alignment may be linked. This is of particular importance when stitching images together that cover different areas with a certain spatial overlay. The temporal alignment within the cardiac cycle might impact the spatial matching, in particular when performed automatically, e.g. error margins might be larger for two images taken at different times of the cardiac cycle than for images taken at the same time of the cardiac cycle. Alternatively or in addition, the registration unit may also exploit data provided by hardware support, e.g. a set of stationary external tracking cameras followed by image processing, a tracking device specifically designed for the ultrasound probe or a transducer-internal tracking device (gyrometer or optoelectronic sensor) which can specify the absolute and/or relative position of an ultrasound probe for each image up to a certain accuracy.

The segmentation unit segments the ultrasound image data to identify the vessel structure. Image segmentation may be performed using a variety of techniques which might be used standalone or in combination to identify meaningful structures of interest in the image data. WO 2016/156446 A1 discloses an ultrasound system and method for identifying vessel structures and is herewith incorporated by reference in its entirety. For the present invention, it is sufficient to obtain local information, preferably 3-D information, a holistic global 3-D model is not required. Optionally, the segmentation unit might use information from the Doppler image data to classify blood pools (areas indicating moving blood) from the surrounding static tissue. Smoothing can preferentially be used in order to compensate for local imaging artifacts. The segmentation of a vessel and the vessel wall can further be improved by edge detection algorithms applied to the ultrasound image data. If the full cross-section of the vessel is not visible in the ultrasound image data, local peak flow velocity values obtained from the Doppler image data can be used to derive the corresponding local cross-sectional area of a vessel and this might further be used for the segmentation.

The representation generation unit generates a representation from the segmented vessel structure. The representation is preferentially a 3D-representation of the vessel structure including at least the major vessels contained in the image data.

A flow velocity determination unit is used to determine flow velocity values from the Doppler image data. Preferentially, static average flow velocity values are determined as the average over a local neighborhood. Depending on the temporal resolution of the Doppler image data, flow velocity values may be determined as values averaged over a cardiac cycle. Alternatively, the flow velocity values may also be determined as a function of time, e.g. over a cardiac cycle, as a flow velocity profile. If the Doppler image data provides input data for more than one cardiac cycle, the flow velocity profile may also provide average flow velocity values for the respective times of the cardiac cycle.

Based on the determined flow velocity values and the representation, the physiological functional parameter determination unit determines the physiological functional parameters using the physiological functional parameter determination model that defines a functional physiological parameter in dependence of a vessel structure representation and flow velocities inside a vessel of the vessel structure. The physiological functional parameter determination model is preferentially a reduced-order functional model and the flow velocity values and the representation are provided as boundary conditions to personalize the model for the living being that is being examined.

The physiological functional parameter is preferentially the intravascular pressure gradient or the (peripheral) fractional flow reserve ((p)FFR). The FFR is commonly used to describe coronary arteries, where the FFR is defined as the ratio of the intravascular pressure after (distal to) a stenosis relative to the pressure of the aorta, e.g. Pd/Pa. Thus, the FFR is an absolute number. Peripheral FFR (p-FFR) is a recent attempt to establish a similar functional index for the peripheral arteries, as suggested inter alia by Issam Koleilat, et al. in "A Novel Simple Technique Using Hyperemia to Enhance Pressure Gradient Measurement of the Lower Extremity During Peripheral Intervention", VASCULAR DISEASE MANAGEMENT 2015; 12(9):E166-E172 which is herewith incorporated by reference. p-FFR is defined by the ratio of the intravascular pressure after (distal to) a stenosis Pd relative to the pressure of a major artery Pa. The pressure gradient is simply the difference between Pa and Pd measured in pressure per length unit.

The functional parameter determination model is preferentially a reduced order functional model, in particular a lumped parameter model, wherein the representation of the vessel structure and the flow velocity values or value profiles extracted from the Doppler image data are used as boundary conditions. The use of lumped parameter models for physiological functional parameter determination is discussed inter alia in "Learning patient-specific lumped models for interactive coronary blood flow simulations" by Hannes Nickisch et al., Medical Image Computing and Computer-Assisted Intervention-MICCAI 2015 (pp. 433-441), Springer International Publishing (2015), which is herewith incorporated by reference. Since reduced order functional model predictions, in particular lumped parameter model predictions, can be computed extremely fast, the results may be provided to sonographers and/or physicians in real-time or at least near-real-time and thus support the diagnosis and treatment of for instance arteriosclerosis.

Preferentially, the apparatus may output the representation as well as the determined physiological functional parameter via a display/monitor wherein the physiological functional parameter may be indicated as color code in the representation of the vessel structure. The display/monitor may be integrally formed with the apparatus or may be an external component connected either wired or wirelessly with the apparatus. Thus, a sonographer conducting the ultrasound probe examination and the person, for instance a physician, viewing the physiological functional parameter and the representation do not have to be the same. They do not even have to be in the same room. A physician might review the examination results from an entirely different location in real-time.

In an embodiment the Doppler image data are first Doppler image data of a first portion of the vessel structure and the determined flow velocity values are first flow velocity values inside a vessel of the first portion of the vessel structure, wherein
the image providing unit is configured to provide second Doppler image data of a second portion of the vessel structure;
the registration unit is configured to register the second Doppler image data with the ultrasound image;
the flow velocity value determination unit is configured to determine second flow velocity values inside a vessel of the second portion of the vessel structure based on the second Doppler image data; and
the physiological functional parameter determination unit is configured to determine the physiological functional parameter using the provided functional parameter determination model, the generated representation of the vessel structure and the determined first and second flow velocity values.

In this embodiment the physiological functional parameter determination can be updated whenever new Doppler image data are provided. The ultrasound image data provided and used to segment the vessel structure may cover several vessels of a vessel structure. Doppler images may be provided for respective positions within the ultrasound image data to determine respective flow velocity values for vessels at these positions. The first Doppler image data may cover a first vessel of the vessel structure covered by the ultrasound image, the second Doppler image data may cover a second vessel of the vessel structure or the first vessel at another location within the vessel structure. The flow velocity values may be fed to the physiological functional parameter determination unit to update the physiological functional parameter determination. The additional flow velocity values may be provided as additional boundary conditions and thus provide a revised physiological functional parameter determination of the entire vessel structure. Newly added Doppler image data may thus also impact the determination of a previously determined physiological functional parameter of another portion of the vessel structure, since the entire model is updated.

In a further embodiment the ultrasound image data are a first ultrasound image data covering a first part of the vessel structure and the generated vessel structure segmentation is a first vessel structure segmentation, wherein
the image providing unit is configured to provide second ultrasound image data of the vessel structure covering a second part of the vessel structure, wherein the second part differs at least partially from the first part;
the registration unit is configured to register the second ultrasound image data with the first ultrasound image data;
the segmentation unit is configured to segment the vessel structure in the second ultrasound image data thereby generating a second vessel structure segmentation; the representation generation unit is configured to generate the representation of the vessel structure based on the first vessel structure segmentation and based on the second vessel structure segmentation.

In this embodiment, the representation of the vessel structure is updated whenever new ultrasound image data are provided. Preferentially, the first and second ultrasound image data have a certain overlap. Then, characteristic features in the overlaying image parts can be used to stitch the image data together. If there is no overlap between the ultrasound image data, e.g. if the ultrasound probe has been discontinuously moved over the skin of the living being or had not continuously taken data, external position information might be provided in order to properly register the first and second ultrasound image data. The representation generation unit updates the representation based on the vessel structure segmented in the first and second ultrasound image data. The additional input can thus be used to update the representation of the vessel structure in real-time. Image registration and segmentation might influence one another and the respective steps may be performed in an iterative manner. The apparatus is preferentially adapted to display the representation of the vessel structure examined with the ultrasound probe and thus provide a live-growing representation whenever a new ultrasound image is provided. Furthermore, since the representation is used as input for the physiological functional parameter determination unit, e.g. as boundary condition of a reduced-order functional model, the further ultrasound image data may also impact the physiological functional parameter determination.

Usually the movement of an ultrasound probe along the skin of a living being will provide both further ultrasound images which extend the coverage area of the vessel structure and further Doppler images which provide further flow velocity measurements. Along with the live-growing representation, e.g. a 3D-model of the vessel structure, the functional parameter determination model is further updated with every new information obtained, e.g. a lumped parameter model is constantly updated with further boundary conditions obtained from the extended representation of the vessel structure as well as the flow velocity values from the Doppler images. Further input data may increase the accuracy of an existing representation as well as the lumped parameter model. Thus, the update of the representation as well as the lumped parameter model may also impact previously determined values of the vessel structure.

In a further aspect of the present invention a method for determining a physiological functional parameter of a living being is presented, the method comprising:
providing ultrasound image data and Doppler image data of a vessel structure of the living being;
registering the ultrasound image data and the Doppler image data;
segmenting the vessel structure in the ultrasound image data;
generating a representation of the vessel structure based on the segmented vessel structure, thereby generating a vessel structure segmentation;
determining flow velocity values inside a vessel of the vessel structure based on the Doppler image data;
providing a functional parameter determination model defining a functional physiological parameter in dependence of a representation of a vessel structure and flow velocity values inside a vessel of the vessel structure;
determining the physiological functional parameter of the living being by using the functional parameter determination model, the generated representation of the vessel structure and the determined flow velocity values.

In an embodiment the Doppler image data are first Doppler image data of a first portion of the vessel structure and the determined flow velocity values are first flow velocity values inside a vessel of the first portion of the vessel structure, and the method further comprises
providing second Doppler image data of a second portion of the vessel structure;
registering the second ultrasound Doppler image data with the first ultrasound image data;
determining second flow velocity values inside a vessel of the second portion of the vessel structure based on the second Doppler image data; and
determining the physiological functional parameter using the provided functional parameter determination model, the generated representation of the vessel structure and the first and second flow velocity values.

In an embodiment the ultrasound image data are first ultrasound image data covering a first part of the vessel structure, wherein the generated vessel structure segmentation is a first vessel structure segmentation and wherein the method further comprises:
providing second ultrasound image data of the vessel structure covering a second part of the vessel structure, wherein the second part differs at least partially from the first part;
registering the second ultrasound image data with the first ultrasound image data;
segmenting the vessel structure in the second ultrasound image data, thereby generating a second vessel structure segmentation;
generating the representation of the vessel structure based on the first vessel structure segmentation and the second vessel structure segmentation.

In a further aspect of the present invention a computer program executable in a processing unit of an apparatus as defined in claim 1 is presented, the computer program comprising program code means for causing the processing unit to carry out a method as defined in claim 10 when the computer program is executed in the processing unit.

It shall be understood that the apparatus for determining a physiological functional parameter of a living being of claim 1, the method for determining a physiological functional parameter of a living being of claim 10, and the computer program for determining a physiological functional parameter of a living being of claim 13 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows an embodiment of an apparatus for determining a physiological functional parameter of a living being;
Fig. 2 schematically and exemplarily shows an embodiment of a method for determining a physiological functional parameter of a living being;
Fig. 3 schematically and exemplarily illustrates a vessel structure derivable with an embodiment of an apparatus or method for determining a physiological functional parameter of a living being;
Fig. 4 schematically and exemplarily shows a lumped parameter model usable to determine a physiological functional parameter a living being; and
Fig. 5 schematically and exemplarily illustrates a measured flow velocity along a vessel and a derived cross-sectional area along the vessel.

### DETAILED DESCRIPTION OF EMBODIMENTS

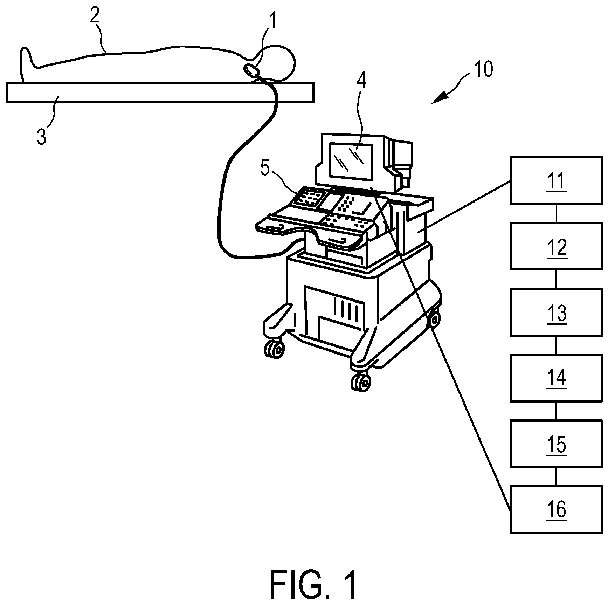

Fig. 1 schematically and exemplarily shows an embodiment of an apparatus 10 for determining a physiological functional parameter of a living being. In this embodiment, the apparatus 10 comprises an ultrasound probe 1 for providing ultrasound image data when being traversed over the skin of a person 2 lying on support means 3. The ultrasound probe 1 can provide both common ultrasound image data as well as Doppler image data wherein the operation mode may be changed manually, e.g. upon pressing a particular button, or automatically, e.g. on a predetermined periodical basis or whenever the probe is moved by a predetermined distance. The ultrasound probe may comprise a tracking device (not shown) specifically designed for the ultrasound probe or a transducer-internal tracking device (gyrometer) (not shown). In order to determine the ultrasound probe's position other hardware may be used, e.g. a set of external tracking cameras followed by image processing, or specific distance sensors for instance integrated in the support means 3. The apparatus 10 further comprises input means 5 which allow the input of specific commands by a user, like start and stop, relevant patient data and/or position data which may optionally be attached to the image data captured with the ultrasound probe 1 for further processing. The ultrasound probe 1 is communicatively coupled to the apparatus 10, either wired or wirelessly. Thus, the ultrasound probe 1 may be within the same room as the apparatus 10 but the apparatus 10 may also be at a completely different location and the ultrasound probe 1 is merely connected to the apparatus 10 via the Internet.

The apparatus 10 has an image providing unit 11 to provide data, preferentially live from the ultrasound probe 1. Alternatively or in addition, the image providing unit 11 may provide images from memory, either stored locally at the apparatus or on a remote server. For instance, in case ultrasound images have already been taken from the person 2, already existing data may be loaded by the image providing unit 11. The image providing unit 11 may also provide partially existing data from memory and add for instance Doppler images for certain positions within the existing pure ultrasound images.

The apparatus 10 further comprises a registration unit 12 for registering the images provided by the image providing unit. With regard to the spatial alignment different sets of data are transformed into one coordinate system. If the ultrasound probe 1 is for instance moved along the skin of the person 2, and a first and second ultrasound image cover a common area of the vessel structure, then similarities in the image data can be used to overlay the images and stitch them together.

However, ultrasound images, in particular Doppler images, may differ not only when the ultrasound probe 1 is moved along the skin of the person 2 and thus the spatial information is changed. The blood flow inside a vessel also fluctuates within a cardiac cycle and an artery or vein might slightly vary in position and/or cross section over a cardiac cycle. If the image data is thus taken once at the peak of a cardiac cycle and once on the lowest point, automatic detection of similarities might require larger error margins to match respective image contours or specific features. Thus, temporal information preferably associated with the cardiac cycle might be used by the registration unit to stitch images together which cover respective first and second parts of a vessel structure having a certain overlay. Furthermore, absolute or relative position data of the ultrasound probe 1 can also be provided to the registration unit 12 to provide a consistent mosaic of ultrasound images and additional Doppler images. Usually ultrasound images are provided in grey scale while Doppler images are overlaid in a color scale, e.g. from blue to red. Usually blue color indicates a flow away from the ultrasound probe 1, and red color indicates a flow towards the ultrasound probe 1. Depending on the chosen resolution, the color might change during a cardiac cycle. When a temporal resolution is required which is sensitive to the cardiac cycle, respective flow profiles across a cardiac cycle are generated. Doppler image data may then comprise a set of Doppler images for respective times within the cardiac cycle. They may be generated from data over one or more cardiac cycles. The flow velocities determined for vessels in the Doppler images may then be provided as flow velocity profiles across a cardiac cycle. If such a temporal resolution is not necessary, static average flow velocity values are determined from the Doppler image data.

Optionally, 3-D ultrasound transducers may be used to gather additional information for the rigid image registration. For instance, in conjunction with an optoelectric sensor (as used in a computer mouse) the trajectory of the transducer can be traced. In order to simplify the image registration, restrictions on the possible trajectories may be imposed by means of a user manual. Registration can be done in the simplest instance by matching image locations using squared distance or correlation measures. Given the information from external or internal tracking devices, the latter could be used to fine-tune the registration.

The apparatus 10 further comprises a segmentation unit 13 for segmenting a vessel structure identified in ultrasound image data. If there is more than one ultrasound image provided by the image providing unit 11, the segmentation unit may also segment a vessel structure from first and second ultrasound images stitched by the registration unit 12. The segmentation unit 13 may use static image features such as the edges of the lumen to segment the vessel structure and/or use dynamic features derived from the Doppler images which for instance indicates flowing blood and thus help to determine the inside of a vessel. The surrounding tissue of a vessel is rather static. The segmentation unit 13 may also allow or request user input to improve or initiate image segmentation. Furthermore, the segmentation unit 13 may smooth the image data provided to compensate for local imaging artifacts. The segmentation unit 13 may also be provided with external input provided by a user via the input means 5 in order to guide the segmentation (a priori) or to correct the segmentation (a posteriori). In order to support the user's assessment, overlays of ultrasound images and the deduced segmentation could be displayed on a display unit 4.

The vessel structure segmented from the aligned ultrasound images is used by a representation generation unit 14 to generate a common representation 200 of the corresponding vessel structure of interest, preferably as a 3D-model. This representation 200 is continuously updated whenever new input data is provided to the segmentation unit, e.g. whenever the ultrasound probe 1 is moved along the skin of the person 2.

Along with the live-growing representation, a physiological functional parameter determination model, e.g. a lumped parameter model 210, is generated and continuously updated which allows to estimate for instance pressure gradients for vessels inside the vessel structure in real-time given a set of suitable boundary conditions. The flow velocity inside a vessel can be determined from the Doppler images and can be used as boundary condition for the lumped parameter model 210. Therefore, the apparatus 10 further comprises a flow velocity determination unit 15 for determining average flow velocities or flow velocity profiles which are provided as boundary conditions to the lumped parameter model 210. Since the Doppler data may only be provided for a portion of the vessel structure, the determination of flow velocities is restricted to these portions. They will preferably capture the most relevant arteries and/or veins in the vessel structure of interest. The apparatus 10 further comprises a physiological functional parameter determination unit 16 for determining pressure gradients or peripheral FFR values using the lumped parameter model 210. Since lumped model predictions can be computed extremely fast, the apparatus 10 may provide real-time or at least near-real-time feedback in clinical practice and thus support sonographers and/or physicians in the diagnosis and treatment of for instance arteriosclerosis. The feedback may be presented in form of the representation 210 which is preferentially color coded according to the determined pressure gradients at the display unit 4 of the apparatus 10 in Fig. 1. Alternatively or in addition, the apparatus 10 may also provide the output to a remote display connected either wired or wirelessly with the apparatus. This way, the person conducting the examination of the person 2 and the person assessing the determined data do not have to be the same. The latter person does not even have to be within the same room. The apparatus 10 may provide the data output for storage, either locally, on hard drive or removable storage, or remote, e.g. at a server or cloud.

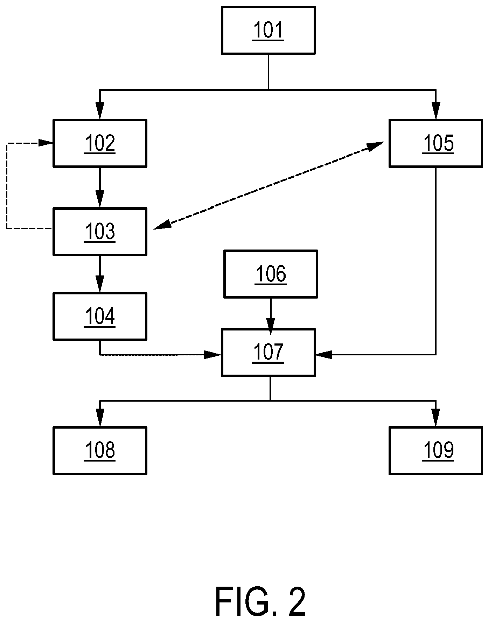

In the following an embodiment of a method for determining a physiological functional parameter of a living being will be exemplarily described with reference to a flow chart shown in Fig. 2.

In step 101 first ultrasound image data covering a first part of the vessel structure of interest are provided, preferentially obtained from an ultrasound probe 1. Furthermore, Doppler image data are provided covering at least a portion of the vessel structure covered by the ultrasound image data. The ultrasound image data are then registered in step 102 with the Doppler image data. In step 103 a vessel structure is segmented from the image data. Step 103 may be performed parallel, subsequent or iteratively with step 102. In case only the ultrasound image data are used for the segmentation, step 103 may be performed independent of step 102. The segmentation comprises basic identification of image structures, such as contours and edges as well as high-level image segmentation specific to structures and patterns typical for the vessel structures. The segmentation step 103 might use further input extracted from the Doppler image data. In that case the previous registration in step 102 is required to merge the information from both image data. In step 104, a representation of vessel structure of interest is generated from the segmented image data. This representation is preferentially a 3D-representation of the main vessels of a vessel structure of interest.

In step 105 flow velocity values are determined from the Doppler image data for respective positions within one or more vessels of a vessel structure. Depending on the temporal resolution average flow velocity values, or flow velocity profiles across a cardiac cycle can be determined. This step may be performed simultaneously to the steps 102 and 103. The Doppler image data may optionally be used for segmentation as indicated by the dashed line between step 105 and step 103. The Doppler image data allow identification of areas with moving blood, in general inside an artery or vein, and the surrounding tissue, which is static and thus does not show the Doppler effect.

In step 107 a physiological functional parameter, e.g. the vascular pressure gradient or the (peripheral) FFR, may be determined for vessels in the representation of the vessel structure using a reduced-order functional model based on determined flow velocities, and the representation of the vessel structure provided in step 106. The determination may use a lumped parameter model wherein the average values of flow velocity, or the flow velocity profiles as well as the representation of the physical vessel structure are used as boundary conditions.

The method may comprise a further step 108 of outputting the representation, preferentially as 3D-model, together with the determined physiological functional parameter values which might be presented in a color-coded manner, wherein specific colors are assigned to predetermined thresholds values of the physiological functional parameter to ease a fast assessment and assist a physician in the diagnosis.

Whenever the ultrasound probe is moved, the method starts again at step 101. Additional image data are provided and registered using internal image information like characteristic patterns as well as external data, cardiac cycle data, position data of the ultrasound probe, etc. The method may also only be fed with further Doppler image data in a region already captured by pure ultrasound image data. In that case, the method continues with step 105 and from there either to step 103, where the Doppler image data are used for segmentation purposes and/or to step 107 to update and extend the lumped parameter model accordingly and determine the physiological functional parameter for the (extended) vessel structure based on the further flow velocity measurements derived from the further Doppler image data. Besides outputting the 3D-representation and the determined physiological functional parameters, the output may also be stored in step 109 in a local or remote storage device, such as a hard drive, optical disc, removable storage medium (USB stick), or on a remote server. Furthermore, the step 108 may comprise transmission of the data via a network to a remote display, such that the person viewing the live-growing anatomical and functional representation does not have to be in the same room as the person 2 examined with the ultrasound probe 1.

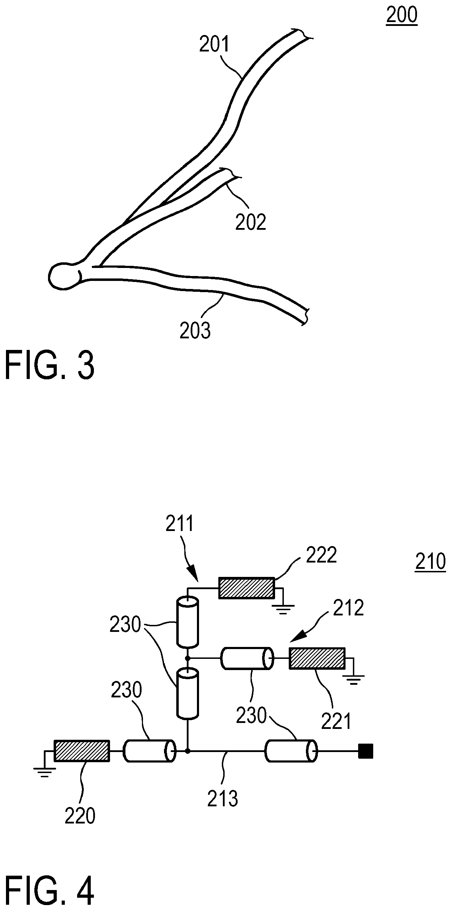

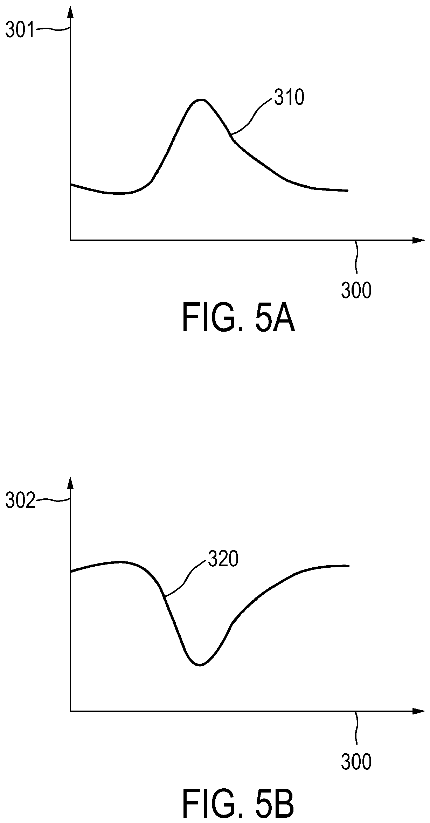

Fig. 3 schematically and exemplarily illustrates a vessel structure derivable from the ultrasound image data. The vessel structure maybe determined using static image features such as the edges of the lumen or dynamic features derivable from Doppler image data which allow to distinguish moving areas, e.g. flowing blood instance areas, e.g. tissue. Additionally or in cases where the full cross section of a vessel is not visible in the ultrasound image data, the local peak flow velocity values 301 derivable from the Doppler image data can be used to derive the local cross sectional area 302 (CSA) as indicated in Figs. 5A and 5B and thus might help to segment the vessel structure.

Fig. 5A shows the flow velocity 301 over the vessel length 300. The resulting curve 310 has the opposite distribution as curve 320, showing the cross sectional area 302 over the vessel length 300. Here a constant blood volume is assumed. Estimating or measuring outflow can further refine this calculation.

The extracted anatomical vessel structure 200 comprising branches 201, 202 and 203 may then be used together with the flow velocity measurements as boundary condition for a reduced-order functional model i.e. a lumped parameter model 210.

Fig. 4 schematically and exemplarily shows a lumped parameter model 210 with three branches 211, 212 and 213 corresponding to branches 201 to 203 of the vessel structure representation 200. The lumped parameter model comprises n = 8 elements and m = 3 nodes including ground. The black boxes 220, 221, 222 indicate inflow and outflow boundary conditions. The white tubes 230 representing tree segment transfer functions are composed of a series of linear and nonlinear resistance elements reflecting both the local vessel geometry and hydraulic effects. Starting from the tree representation shown in Fig. 3, a circuit with two macroscopic component types is set up: nonlinear vessel segment resistors 230 and boundary conditions 220, 221, 222. The boundary condition may be a pressure or flow source driving the network; but any (lumped) boundary condition driving a conventional finite element model can be used here. Methods how to translate the local geometry of the vessel (radius, perimeter, cross-sectional area) into parameters of the nonlinear resistor are well known and disclosed, for instance, in the article "Learning patient-specific lumped models for interactive coronary blood flow simulations" by Hannes Nickisch et al. cited herein above.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An apparatus (10) for determining a physiological functional parameter of a living being (2), the apparatus (10) comprising:
an image providing unit (11) for providing ultrasound image data and Doppler image data of a vessel structure of the living being (2);
a registration unit (12) for registering the ultrasound image data and the Doppler image data;
a segmentation unit (13) for segmenting the vessel structure in the ultrasound image data, thereby generating a vessel structure segmentation;
a representation generation unit (14) for generating a representation (200) of the vessel structure based on the vessel structure segmentation;
a flow velocity determination unit (15) for determining flow velocity values inside a vessel of the vessel structure based on the Doppler image data; and
a physiological functional parameter determination unit (16) for determining the physiological functional parameter of the living being (2), wherein the physiological functional parameter determination unit is adapted to a) provide a functional parameter determination model (210) defining a functional physiological parameter in dependence of a representation of a vessel structure and flow velocity values inside a vessel of the vessel structure, and b) determine the physiological functional parameter by using the functional parameter determination model, the generated representation of the vessel structure and the determined flow velocity values.

2. The apparatus (10) according to claim 1, wherein
the Doppler image data are first Doppler image data of a first portion of the vessel structure and the determined flow velocity values are first flow velocity values inside a vessel of the first portion of the vessel structure;
the image providing unit (11) is configured to provide second Doppler image data of a second portion of the vessel structure;
the registration unit (12) is configured to register the second Doppler image data with the ultrasound image data;
the flow velocity value determination unit (15) is configured to determine second flow velocity values inside a vessel of the second portion of the vessel structure based on the second Doppler image data; and
the physiological functional parameter determination unit (16) is configured to determine the physiological functional parameter using the provided functional parameter determination model (210), the generated representation of the vessel structure and the determined first and second flow velocity values.

3. The apparatus (10) according to claim 1, wherein the ultrasound image data are first ultrasound image data covering a first part of the vessel structure, wherein the generated vessel structure segmentation is a first vessel structure segmentation and wherein
the image providing unit (11) is further configured to provide second ultrasound image data of the vessel structure covering a second part of the vessel structure, wherein the second part differs at least partially from the first part;
the registration unit (12) is configured to register the second ultrasound image data with the first ultrasound image data;
the segmentation unit (13) is configured to segment the vessel structure in the second ultrasound image data, thereby generating a second vessel structure segmentation;
the representation generation unit (14) is configured to generate the representation (200) of the vessel structure based on the first vessel structure segmentation and based on the second vessel structure segmentation.

4. The apparatus (10) according to claim 1, wherein the registration unit (12) comprises a position detection unit providing position data of an ultrasound probe (1) used to generate the ultrasound image data and the Doppler image data, wherein the registration unit (12) is adapted to use the position data for registering the ultrasound image data and the Doppler image data.

5. The apparatus (10) according to claim 1, wherein the segmentation unit (13) is configured to identify moving and static areas in the Doppler image data and to segment the vessel structure based at least in part on the identified moving and static areas.

6. The apparatus (10) according to claim 5, wherein the segmentation unit (13) is configured to determine local peak flow values for a vessel of the vessel structure in the moving areas of the Doppler image data, to determine cross-sectional areas of the vessel based on the local peak flow values and to segment the vessel structure in the ultrasound image data by using the cross-sectional areas.

7. The apparatus (10) according to claim 5, wherein the segmentation unit (13) is further configured to segment the vessel structure in the ultrasound image data by using a lumen edge detection algorithm.

8. The apparatus (10) according to claim 1, wherein the physiological functional parameter is a vascular pressure gradient or a peripheral fractional flow reserve.

9. The apparatus (10) according to claim 1, wherein the functional parameter determination model is a reduced order functional model.

10. A method for determining a physiological functional parameter of a living being (2), the method comprising:
providing (101) ultrasound image data and Doppler image data of a vessel structure of the living being (2);
registering (102) the ultrasound image data and the Doppler image data;
segmenting (103) the vessel structure in the ultrasound image data;
generating (104) a representation (200) of the vessel structure based on the segmented vessel structure, thereby generating a vessel structure segmentation;
determining (105) flow velocity values inside a vessel of the vessel structure based on the Doppler image data;
providing (106) a functional parameter determination model (210) defining a functional physiological parameter in dependence of a representation of a vessel structure and flow velocity values inside a vessel of the vessel structure;
determining (107) the physiological functional parameter of the living being (2) by using the functional parameter determination model (210), the generated representation of the vessel structure and the determined flow velocity values.

11. The method according to claim 10, wherein the Doppler image data are first Doppler image data of a first portion of the vessel structure and the determined flow velocity values are first flow velocity values inside a vessel of the first portion of the vessel structure, the method further comprising:
providing (101) second Doppler image data of a second portion of the vessel structure;
registering (102) the second Doppler image data with the ultrasound image data;
determining (104) second flow velocity values inside a vessel of the second portion of the vessel structure based on the second Doppler image data; and
determining (105) the physiological functional parameter using the provided model (210), the generated representation of the vessel structure and the first and second flow velocity values.

12. The method according to claim 10, wherein the ultrasound image data are first ultrasound image data covering a first part of the vessel structure, wherein the generated vessel structure segmentation is a first vessel structure segmentation and wherein the method further comprises:
providing second ultrasound image data of the vessel structure covering a second part of the vessel structure, wherein the second part differs at least partially from the first part;
segmenting the vessel structure in the second ultrasound image data, thereby generating a second vessel structure segmentation;
generating the representation (200) of the vessel structure based on the first vessel structure segmentation and the second vessel structure segmentation.

13. A computer program for determining a physiological functional parameter of a living being (2) executable in a processing unit of an apparatus (10) as defined in claim 1, the computer program comprising program code means for causing the processing unit to carry out a method as defined in claim 10 when the computer program is executed in the processing unit.
